# EUROPEAN PATENT SPECIFICATION

(11) **EP 3 275 425 B1**
(45) Date of publication and mention of the grant of the patent: **03.03.2021**
(21) Application number: 16181292.0
(22) Date of filing: 26.07.2016
(51) Int. Cl.: A61K 8/34, A61Q 5/06, A61K 8/81, A61K 8/898

(54) **HAIR STYLING COMPOSITION**
HAARSTYLINGZUSAMMENSETZUNG
COMPOSITION DE COIFFURE

(43) Date of publication of application: 31.01.2018
(73) Proprietor: Kao Germany GmbH, 64297 Darmstadt (DE)
(72) Inventor: Schmid, Sabine, 64297 Darmstadt (DE); Sulzbach, Melina, 64297 Darmstadt (DE)
(74) Representative: Grit, Mustafa

(56) References cited:
- WO-A1-2009/103735
- US-A1- 2010 047 202
- US-A1- 2014 099 275
- DATABASE GNPD [Online] MINTEL; 1 June 2013 (2013-06-01), Innovations Scotland: "Mega Hold Hair Styling Gel", XP002763089, Database accession no. 2108583
- "Fixate(TM) Freestyle Polymer - TECHNICAL DATA SHEET", , 11 December 2014 (2014-12-11), XP055231469, Retrieved from the Internet: URL:https://www.lubrizol.com/Personal-Care /Documents/Technical-Data-Sheets/TDS-773-F ixate(TM)-Freestyle-Polymer.pdf [retrieved on 2015-11-26]

## Description

The present invention is on a composition to style keratin fibers, preferably human keratin fibers, more preferably human hair.

A large percentage of the world's population has natural curly hair which is often unruly, exhibits a rough surface, and possesses a certain degree of frizz. Moreover, the appearance of the curls is strongly dependent on environmental conditions such as humidity and temperature. Under certain conditions customers with curly hair are not able to manage or style their hair at all and have to rely on chemical processes to enhance manageability and styling of the hair.

Such styling products that claim to reduce frizz are known to the one skilled in the art which comprise relaxing ingredients such as strong alkaline compounds (e.g. Mintel 3357289) that confer the hair an undesired texture by flattening the hair on the scalp and leaving a rough surface structure on the hair.

Besides chemical relaxers, there is a great number of hair styling gels on the market which are claimed to have a high degree of hold (Mintel 2282179, Mintel 1895066, Mintel 3673285, Mintel 2260201, Mintel 2009811). Reference is also made to WO 2009/103735 A1.

Especially for clients with fine and limp curls it is challenging to find a styling product that supports the natural shape and enhances the style/curl retention. The literature is silent on delivering a composition with such properties. Consequently there is a great need for hair styling products that smooth/calm the surface of hair without showing the aforementioned flattening effect on hair.

It was surprisingly found that a composition comprising film-forming polymers, polyols, and aminosilicones provides great styling benefits for customers with curly hair without showing the aforementioned flattening effect.

Consequently, the first object of the present is a cosmetic composition characterized in that it comprises
a) a film forming polymer prepared with the monomers comprising acrylic acid, methacrylic acid and/or methacrylic acid esters, wherein the crosslinkers are selected from glycol dimethacrylate, trimethylolpropane triacrylate, trimethylolpropane diallyl ether, individually or in any mixture thereof,
b) one or more polyols at a concentration from 8% to 18% by weight, calculated to the total of the composition,
c) one or more aminosilicones,
wherein the composition comprises one or more additional film-forming polymer(s) selected from copolymers or homopolymers of polyvinylpyrrolidone.

The second object is the use of the composition for styling keratin fibers, preferably human keratin fibers, more preferably human hair and for reducing the frizziness of hair.

The third object is a method to style hair wherein the composition of the present invention is applied onto keratin fibers, preferably human keratin fibers, more preferably human hair.

Further object of the present invention is a kit for hair comprising the composition of the present invention.

The composition of the present invention comprises a film forming polymer prepared with the monomers comprising acrylic acid, methacrylic acid and/or methacrylic acid esters, wherein the crosslinkers are selected from glycol dimethacrylate, trimethylolpropane triacrylate, trimethylolpropane diallyl ether, individually or in any mixture thereof. Such polymers are disclosed in EP2611412 by Lubrizol Corporation. The polymers are also known under their CTFA names acrylates crosspolymer, and examples are acrylates crosspolymer, acrylates crosspolymer-3, and acrylates crosspolymer-4.

The total concentration of the acrylates cross polymers in the composition is in the range from 0.01% to 10% by weight, preferably from 0.1% to 7.5% by weight, more preferably from 0.5% to 6% by weight, and most preferably from 1% to 5% by weight, calculated to the total of the composition.

The composition comprises one or more additional film-forming polymers selected from copolymers or homopolymers of polyvinylpyrrolidone. Homopolymers of vinylpyrrolidone are available at various molecular weight ranges from BASF Corporation under the trade names Luviskol K.

Typical molecular weight ranges for polyvinylpyrrolidones suitable for the present invention range from 10 kDa up to 200 kDa, preferably from 20 kDa to 100 kDa, more preferably from 25 kDa to 100 kDa, wherein the molecular weight ranges are given as average molecular weight ranges.

The composition may further comprise copolymers of polyvinylpyrrolidine. Suitable copolymers comprise units of vinylpyrrolidone and at least one more co-monomer selected from vinylacetate, styrene, vinylpyridine, and vinylimidazole. The composition may comprise a poly-(vinylpyrrolidone-co-vinylacetate) copolymer wherein the ratio of comonomers can be varied ranging from VP/VA 30:70 to VP/VA 70:30. Such polymers have a typical average molecular weight ranging from 20 kDa to 40 kDa and are offered for sale by BASF Corporation under the trade name Luviskol VA.

The preferred additional film-forming polymer is polyvinylpyrrolidone which is a homopolymer of vinylpyrrolidone.

The weight ratio of the compound a) to the additional film forming polymer is in the range from 1 to 10, more preferably from 2 to 8, and most preferably from 2 to 6. The composition of the present invention may further comprise natural non-ionic polymers. Those are for example cellulose, chitosan, guar gum, neutralised shellac and their derivatives.

The composition of the present invention comprises one or more polyols. The term polyol is to be understood as a molecule comprising two or more hydroxyl groups and having a linear carbon chain length from C2 to C8 or branched carbon chain length from C3 to C8.

Suitable polyols are ethylene glycol, diethylene glycol, propylene glycol, dipropylene glycol, butylene glycol, dibutylene glycol, glycerol, panthenol and its derivatives. It is further suitable to use mixtures of polyols. Preferaply the composition comprises glycerine and more preferably glycerine is the sole polyol.

The total concentration of polyols is in the range from 8% to 18% by weight, calculated to the total of the composition.

The composition comprises one or more aminosilicones wherein the term aminosilicone is to be understood as an aminated silicone comprising at least one primary, secondary, tertiary, and/or quaternary amino group and is referred to as amodimethicone or aminodimethicone. The aminosilicone is preferably selected from:
(1) a compound according to the general structure 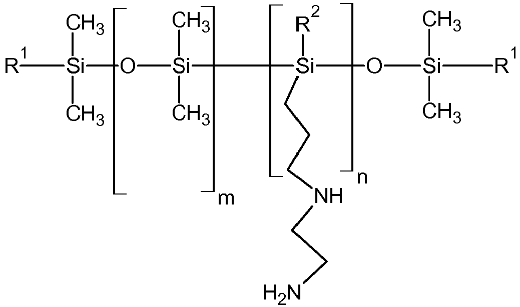 Wherein R¹ is selected from OH, OCH₃, and/or O-Si-(CH₃)₃, R² is selected from CH₃, OCH₃, O-(Si-(CH₃)₂)ₓ-R¹, and/or O-Si-(CH₃)₃, with the provision that if R¹ or R² are selected from O-Si-(CH₃)₃, then all other R¹ or R² are selected from O-Si-(CH₃)₃ and/or OCH₃.
(2) silicone graft copolymer comprising an organopolysiloxane segment as a main chain thereof and an unsaturated monomer-derived polymer segment as a side chain thereof, which is obtainable by firstly reacting an aminopropyl dimethicone with the thiolactone of acetyl homocysteine and then graft copolymerizing the thus obtained mercapto modified dimethicone with a mixture of N,N-dimethylacrylamide and N-t-butylacrylamide. Such a polymer is known under the CTFA name Polysilicone 28 and marketed by Kao Corporation.
(3) an organopolysiloxane, wherein at least two silicone atoms in an organopolysiloxane segments constituting a main chain of the organopolysiloxane are bound to poly(N-acylalkyleneimine) segments consisting of repeating units represented by the following general formula via alkylene group containing hetero atom: 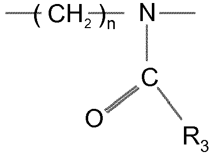 wherein R3 is a hydrogen atom, an alkyl group having 1 to 22 carbon atoms, an aralkyl group, or an aryl group; and n is 2 or 3; wherein the number-average molecular weight of the poly(N-acylalkyleneimine) segment is from 1,200 to 5,500, wherein the mass ratio of the organopolysiloxane segments (a) constituting the main chain to the poly(Nacylalkyleneimine) segments (b) i.e., a/b is from 35/65 to 60/40, wherein the weight-average molecular weight of the adjacent poly(N-acylalkyleneimine) segments is from 1,300 to 5,500, and wherein the weight-average molecular weight of the organopolysiloxane segment constituting the main chain is from 7,000 to 100,000. Such a polymer is known under the CTFA name Polysilicone 9 and marketed by Kao Coporation. Derivatives of this polymer are disclosed in EP2502615.

The total concentration of silicone compounds is in the range from 0.01% to 7.5% by weight, preferably from 0.05% to 5% by weight, and more preferably from 0.1% to 4%, further more preferably from 0.2% to 3% by weight, calculated to the total of the composition.

The composition is an aqueous composition with water content of more than 50% by weight, calculated to the total of the composition. The composition may comprise further organic solvents with a concentration of up to 25% by weight, calculated to the total of the composition, other than polyols. Suitable are ethanol, n-propanol, and n-butanol, iso-propanol, tert-butanol, iso-butanol, phenol, phenoxyethanol, benzyl alcohol, 2-phenylethanol, and 2-benzoyloxyethanol. The skilled in the art will recognize that some of the aforementioned organic solvents can act as preservatives.

The composition of the present invention may further comprise surfactants selected from non-ionic, cationic, anionic or zwitterionic surfactants, or mixtures thereof.
Suitable non-ionic surfactants are long-chain fatty acid mono- and dialkanolamides, such as coco fatty acid mono- or diethanolamide and myristic fatty acid mono or diethanolamide, stearic acid mono or diethanolamide, alkyl polyglucosides with an alkyl group of 8 to 18 carbon atoms, and with 1 to 5 glucoside units, poly-condensates of ethyleneoxide and propyleneoxide, as they are on the market, for example, under the trade name "Pluronics^{R}". Suitable non-ionic surfactants are esters or ethers of ethylene oxide and fatty acids or fatty alcohols, such as ethoxylated castor oil, ethoxylated coconut fatty acid, ethoxylated lauric acid, ethoxylated oleic acid, ethocylated stearic acid, sorbitan esters, such as polyethylene glycol sorbitan stearic, palmitic, myristic and lauric acid esters, fatty acid polyglycol esters or, as well as fatty alcohol ethoxylates, C₁₀-C₂₂-fatty alcohol ethoxylates, known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16": The average degree of ethoxylation thereby ranges between about 2.5 and about 100, preferably about 10 and about 30.

Anionic surfactants suitable are in principle known from the cleansing compositions. These are anionic surfactants of the sulfate, sulfonate, carboxylate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof as well as alkyl amido polyether carboxylic acids and salts thereof. Such products have been known for some time and are on the market, for example, under the trade name "AKYPO®" and "AKYPO-SOFT®".

Also useful are C₈-C₂₀-acyl isethionates, alone or in admixture with other anionic surfactants, as well as sulfofatty acids and the esters thereof.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.
It is also possible to use mixtures of several anionic surfactants.

Suitable zwitterionic surfactants are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and - acetate have also proven suitable.

Suitable cationic surfactants are according to the general structure 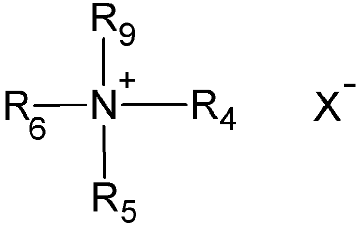 where R₅ is a saturated or unsaturated, branched or linear alkyl chain with 8-22 C atoms or

R₇CONH(CH₂)ₙ

where R₇ is saturated or unsaturated, branched or linear alkyl chain with 7-21 C atoms and n has typical value of 1 - 4 or

R₈COO(CH₂)ₙ

where R₅ is saturated or unsaturated, branched or linear alkyl chain with 7-21 C atoms and n has typical value of 1 - 4, and
R₄ is H or unsaturated or saturated, branched or linear alkyl chain with 1 - 22 C atoms or

R₇CONH(CH₂)ₙ

or

R₈COO(CH₂)ₙ

where R₇, R₈ and n are same as above.

R₉ and R₆ are H or lower alkyl chain with 1 to 4 Carbon atoms, and X is typically chloride, bromide, methosulfate.

Typical examples of those ingredients are cetyl trimethly ammonium chloride, stearyl trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

The preferred surfactant type is non-ionic surfactants which can be employed in mixtures within the group of non-ionic surfactants or in mixture with any other type of surfactant selected from anionic, cationic, or zwitterionic. The total concentration of one or more surfactants in the composition ranges from 0.1% to 20%, preferably 0.2% to 15% and most preferably 0.2% to 10% by weight, calculated to the total of the composition.
The composition may further comprise one or more ceramide compound, such as the one according to general formula 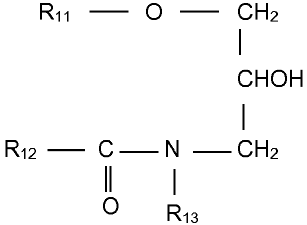 where R₁₁ and R₁₂ are independent from each other alkyl- or. alkenyl group with 10 to 22 carbon atoms, R₁₃ is alkyl or hydroxyl alkyl with 1 to 4 carbon atoms group and n is a number between 1 to 6, preferably 2 or 3. The preferred compound according to the above chemical structure is cetyl-PG-hydroxyethylpalmitamide. Concentration of ceramide type of compounds ranges from 0.01% to 2%, preferably 0.01% to 1% by weight calculated to the total of the composition.

The composition may further comprise ubiquinone of the formula: 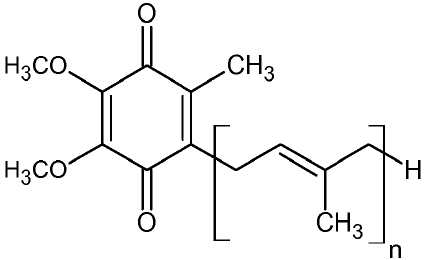 wherein n is a number from 1 to 10. The concentration of ubiquinone can vary between 0.001% and 10% by weight, calculated to the total of the composition.

The composition may comprise oils from vegetable origin. Vegetable C₁₀- to C₃₆-fatty acid triglycerides are for example castor oil, coconut oil, corn oil, cottonseed oil, olive oil, palm kernel oil, peanut oil, rapeseed oil, sunflower oil, safflower oil, sesame oil, soybean oil, almond oil, cashew oil, hazelnut oil, jojoba oil, macadamia oil, pecan oil, pine nut oil, pistachio oil, walnut oil, grapefruit seed oil, lemon oil, orange oil, pumpkin seed oil, flaxseed oil, apricot kernel oil, argan oil, avocado oil, babassu oil, cocoa butter, grape seed oil, mustard oil, poppyseed oil, prune kernel oil, rice bran oil, and what germ oil.

The composition may comprise fatty alcohols are the ones with a carbon chain length of 14 to 22 C atoms which may be saturated or unsaturated, linear or branched which may as well be substituted. Non-limiting examples are myristyl alcohol, cetyl alcohol, stearyl alcohol, behenyl alcohol and cetostearyl alcohol, oleyl alcohol, as well as their mixtures.

The composition may comprise waxes which are esters of fatty acids with fatty alcohols wherein at least one of the fatty components of the ester has a carbon chain length from C₁₀ to C₂₂. Suitable waxes are beeswax, lanolin, carnauba wax, candelilla wax, ouricury wax, cetyl palmitate, isopropyl plamitate, octyl palmitate, isocetyl palmitate, and octyl stearate, myristyl myristate, lauryl palmitate, as well as their mixtures. Further suitable waxes are diesters of adipic acid with mixed diesters of caprylic, capric, hydroxystearic, and isostearic acid which are available under the CTFA names bis-diglyceryl polyacyladipate-1 and bis-diglyceryl polyacyladipate-2, as well as their mixtures. The preferred waxes are bis-diglyceryl polyacyladipate-1 and bis-diglyceryl polyacyladipate-2.

The composition may comprise petrolatum-based products are linear and/or branched paraffins with a carbon chain length of C₆ to C₁₂, mineral oils, preferably light mineral oils, and petroleum jelly, as well as their mixtures.

The total concentration of oil from vegetable origin, fatty alcohols and waxes as well as petrolatum-based products is limited to a maximum of 5%, preferably 2.5% and more preferably 1% by weight, calculated to the total of the composition.

The aqueous composition of the present invention may further comprise one or more UV filters which may be selected from water soluble ones as well as oils soluble ones. The oil soluble UV filter are more preferred ones as they show no interaction with the cationic quaternary ammonium polymers. Non-limiting examples are 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene)-DL-campher, and/or polysilicone-15.

The total UV filter concentration may be in the range of 0.01% to 2.5 % by weight, calculated to the total of the composition.

The composition may comprise essential oils such as angelica essential oil, anise essential oil, basil essential oil, bergamot essential oil, bitter almond essential oil, black pepper essential oil, boldo essential oil, calamus essential oil, camomile essential oil, cardamom essential oil, carrot seed essential oil, cassia essential oil, cedarwood essential oil, cinnamon essential oil, citronella essential oil, coriander essential oil, cypress essential oil, dill essential oil, eucalyptus essential oil, fennel essential oil, frankinscense essential oil, geranium essential oil, ginger essential oil, grapefruit essential oil, helicrysum essential oil, jasmine essential oil, juniper essential oil, lavandin essential oil, lavender essential oil, lemon essential oil, lemongrass essential oil, lime essential oil, mandarin essential oil, melissa essential oil, mullein essential oil, myrtle essential oil, orange essential oil, oregano essential oil, peppermint essential oil, pine essential oil, rose essential oil, rosemary essential oil, rosewood essential oil, sandalwood essential oil, spearmint essential oil, tangerine essential oil, tea tree essential oil, thuja essential oil, vanilla essential oil, wintergreen essential oil, ylang ylang essential oil. The skilled in the art will recognize that some of the aforementioned essential oils can be employed as fragrance. However, the composition of the present invention may comprise a conventional fragrance as well.

The total concentration of essential oils is less than 1% by weight, calculated to the total of the composition.

The composition of the present invention may further comprise natural plant extracts. Within the meaning of the present invention the extracts are liquid extracts and prepared by mixing plant parts such as leaves, fruits, blossoms and roots with a solvent such as water, alcohol, propyleneglycol or mixture of more than one solvent and incubating for certain period of time and filtrating the undissolved plant parts. Suitable aqueous (e.g. steam-distilled) alcoholic or hydroalcoholic plant extracts known per se are in particular aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, the various "Extrapon" products, "HerbasolR", "SedaplantR" and "HexaplantR". Extracts and the preparation thereof are also described in "Hagers Handbuch der pharmazeutischen Praxis", 4th Ed. Preferred plant extracts are prepared from Vitis vinifera, Malus domestica, Camelia sinensis, Juglans regia Ribes Uva-Crispa, Ribes nigrum, Ribes rubrum and Punica granatum. The above mentioned extracts may also be available in the powder form and such are also suitable within the meaning of the present invention.
The natural plant extracts are included into the compositions at a concentration of 0.001 to 1.0%, preferably 0.005 to 0.75%, more preferably 0.01 to 0.6% and most preferably 0.05 to 0.5% by weight, calculated to the total of the composition based on dry matter of the extract.

The composition may comprise vitamins and/or their derivatives such as vitamins A, B group, C, D group, E group, and K. It may further comprise antioxidants such as green tea extract, emblica extract, rosemary extract, and ginkgo extract.
The composition may further comprise proteins or hydrolyzed proteins such as keratin, elastin, collagen, or the ones generated from wheat, barley, quinoa, rye, rice, milk, amaranth, hazelnut, corn, soybean, avocado, brazil nut, casein, cottonseed, egg, honey, jojoba, oat, potato, royal jelly, sesame, silk, sweet almond, whey, and yeast. Proteins or protein hydrolyzates are comprised at a concentration in the range from 0.01% to 5%, preferably 0.1% to 3% and more preferably 0.2% to 2.5% and most preferably 0.25% to 2% by weight calculated to the total of the composition.
In another embodiment of the present invention the composition comprise one or more hair direct dyes. Suitable ones are cationic, anionic and nitro dyes. Plant dyes are also suitable for the compositions of the present invention.

Suitable anionic direct dyes are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium. Among those, the most preferred anionic dyestuffs are Acid Red 52, DC Violet 2, DC Red 33, DC Orange 4, DC Red 27, DC Yellow 10, HC Blue 18, HC Red 18, and HC Yellow 16.

Suitable cationic dyes are in principle those available on the market for cosmetic hair colouring applications. For this purpose, special reference is made to the PCT application WO 95/15144 of Ciba-Geigy AG. Some examples to those are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic red 51, Basic Yellow 87, HC Blue 17 and Basic Orange 31. The most preferred ones are Basic red 51, Basic Yellow 87 and Basic Orange 31 sold by BASF, and HC Blue 17.
Suitable nitro dyes are HC Blue No.2, HC Blue No.4, HC Blue No.5, HC B0lue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder.

The composition may comprise one or more hair direct dye at a total concentration of 0.01% to 5%, preferably 0.05% to 4% and more preferably 0.1% to 2.5% by weight calculated to the total of the composition. The composition can also comprise a mixture of several direct dyes, i.e., an anionic, a cationic and/or nonionic ones. In such a case the dyes may be mixed at any ratio with each other.

The composition may further comprise any known preservatives if necessary.

The following examples are to illustrate the invention, but not to limit it.

### EXAMPLES

The following compositions were prepared by conventional dissolution and mixing techniques.

| | **Inventive 1** | **Inventive 2** | **Inventive 3** | **Comp. A** | **Comp. B** | **Comp. C** | **Comp. D** | **Comp. E** | **Comp.F** |
|---|---|---|---|---|---|---|---|---|---|
| | % [w/w] | | | | | | | | |
| **Glycerol 99%** | 15 | 15 | 15 | 25 | 20 | 20 | 20 | 20 | 20 |
| **Acrylates Cross polymer** | 3 | - | - | - | - | - | - | - | - |
| **Acrylates Crosspolymer-3** | - | 3 | | 3 | 3 | 1 | 4 | - | - |
| **Acrylates Crosspolymer-4** | - | - | 3 | - | - | - | - | - | 3 |
| **PVP** | 1 | 1 | 1 | 1 | 1 | 3 | - | 4 | - |
| **Polysilicone 28** | 0.5 | 0.5 | 0.5 | 0.5 | 0.5 | 0.5 | 0.5 | 0.5 | 0.5 |
| **Water** | ad 100 | | | | | | | | |

Human hair streaks (20 cm long, Caucasian hair) were purchased from International Hair Products Inc., Glendale, NY, USA. The hair streaks were curled by a conventional perming process on curlers. The perming compositions are available on the market under the brand name Goldwell Straight'N Shine. The permed hair streaks were photographed from a pre-set distance. Then the streaks were treated with 2 g of either the inventive composition A or the comparative compositions A-F. The compositions were sprayed onto the hair by atomizing without a propellant. The compositions were then allowed to air dry for 1 h at room temperature.

Each hair streak was placed in a freely-hanging fashion and combed through for three times with a conventional hair dresser comb. Photos for each hair streak were taken from the same distance as before in the permed state. Then the photos were analyzed by a proprietary image analysis software by calculating the occupied area of the hair streak in the image based on the grey scale contrast differences between the hair streaks and the background. The area was recorded as cm² and the increase of area upon combing was calculated. Lower increase values in % correspond to higher curl retention.

| **Measured Tresses** | | | | | | | |
|---|---|---|---|---|---|---|---|
| | Inventive 1 | Comp. A | Comp. B | Comp. C | Comp. D | Comp. E | Comp. F |
| Area before treatment with compositions [cm²] | 9,1 | 8,2 | 10,1 | 9,9 | 11,2 | 9,2 | 8,6 |
| Area upon combing [cm²] | 16,7 | 22,5 | 27,4 | 26,5 | 49,7 | 24,6 | 48 |
| Difference [cm²] | 7,6 | 14.3 | 17,3 | 16,6 | 38,5 | 15,4 | 39,4 |
| **Increase [%]** | **183,5** | **274,4** | **271,3** | **267,7** | **443,8** | **267,4** | **558,1** |

As evident from the tests above, the curl retention of the inventive composition 1 was much higher compared the comparative compositions. Neither acrylates crosspolymer-3 alone nor PVP alone were able to achieve that effect. Also a different ratio of acrylates crosspolymer-3 and PVP was not able to solve the problem (comparative composition C). Consequently the inventive composition was proven superior in comparison to all comparative compositions.

## Claims

1. A cosmetic composition **characterized in that** it comprises
a) a film forming polymer prepared with the monomers comprising acrylic acid, methacrylic acid and/or methacrylic acid esters, wherein the crosslinkers are selected from glycol dimethacrylate, trimethylolpropane triacrylate, trimethylolpropane diallyl ether, individually or in any mixture thereof, preferably at a concentration in the range of 0.01 to 10% by weight, calculated to the total of the composition,
b) one or more polyols at a concentration from 8% to 18% by weight, calculated to the total of the composition,
c) one or more aminosilicones,
wherein the composition comprises one or more additional film-forming polymer(s) selected from copolymers or homopolymers of polyvinylpyrrolidone.

2. Composition according to claims 1 and/or 2 **characterized in that** film-forming polymer is a homopolymer of polyvinylpyrrolidone.

3. Composition according to any of the preceding claims **characterized in that** the weight ratio to the compound of a) and the additional film forming polymer is in the range from 1 to 10, more preferably from 2 to 8, and most preferably from 2 to 6.

4. Composition according to any of the preceding claims **characterized in that** the polyols are selected from molecules comprising 2 or more hydroxyl groups and having a linear carbon chain length from C2 to C8 or branched carbon chain length from C3 to C8.

5. Composition according to any of the preceding claims **characterized in that** the composition comprises glycerine as the polyol, preferably as the sole polyol.

6. Composition according to any of the preceding claims **characterized in that** the aminosilicone is selected from:
a compound according to the general structure 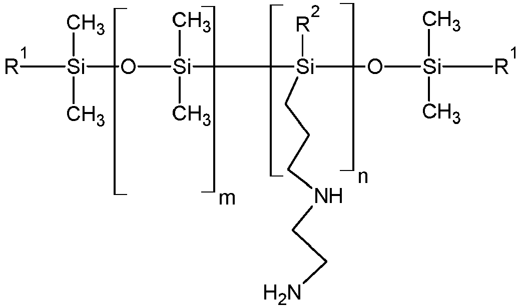 Wherein R¹ is selected from OH, OCH₃, and/or O-Si-(CH₃)₃, R² is selected from CH₃, OCH₃, O-(Si-(CH₃)₂)ₓ-R¹, and/or O-Si-(CH₃)₃, with the provision that if R¹ or R² are selected from O-Si-(CH₃)₃, then all other R¹ or R² are selected from O-Si-(CH₃)₃ and/or OCH₃.
b. silicone graft copolymer comprising an organopolysiloxane segment as a main chain thereof and an unsaturated monomer-derived polymer segment as a side chain thereof, which is obtainable by firstly reacting an aminopropyl dimethicone with the thiolactone of acetyl homocysteine and then graft copolymerizing the thus obtained mercapto modified dimethicone with a mixture of N,Ndimethylacrylamide and N-t-butylacrylamide,
c. an organopolysiloxane, wherein at least two silicon atoms in an organopolysiloxane segments constituting a main chain of the organopolysiloxane are bound to poly(N-acylalkyleneimine) segments consisting of repeating units represented by the following general formula (2) via alkylene group containing hetero atom: 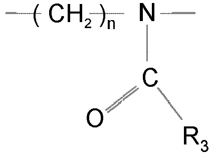 wherein R3 is a hydrogen atom, an alkyl group having 1 to 22 carbon atoms, an aralkyl group, or an aryl group; and n is 2 or 3; wherein the number-average molecular weight of the poly(N-acylalkyleneimine) segment is from 1,200 to 5,500, wherein the mass ratio of the organopolysiloxane segments (a) constituting the main chain to the poly(Nacylalkyleneimine) segments (b) [Le., alb] is from 35/65 to 60/40, wherein the weight-average molecular weight of the adjacent poly(N-acylalkyleneimine) segments is from 1,300 to 5,500, and wherein the weight-average molecular weight of the organopolysiloxane segment constituting the main chain is from 7,000 to 100,000.

7. Composition according to any of the preceding claims **characterized in that** it is an aqueous composition with a concentration of less than 25% by weight of organic solvents other than polyols, preferably with less than 20% by weight other than polyols, and more preferably with less than 15% by weight other than polyols, calculated to the total of the composition.

8. Composition according to any of the preceding claims **characterized in that** it comprises one or more surfactants selected from non-ionic, cationic, anionic or zwitterionic or mixtures thereof.

9. Composition according to claim 9 **characterized in that** it comprises one or more non-ionic surfactants.

10. Composition according to any of the preceding claims **characterized in that** it comprises one or more of the compounds selected from UV filters, antioxidants, preservatives, fragrance, ceramides, ubiquinones.

11. Composition according to any of the preceding claims characterized that the viscosity of the composition is above than 10,000 mPas measured with a Brookfield viscosimeter at 25°C.

12. Use of the composition according to claims 1 to 11 for styling keratin fibers, preferably human keratin fibers, more preferably human hair and/or for reducing the frizziness of hair.

13. Method to style hair **characterized in that** the composition defined in the claims 1 to 11 is applied onto keratin fibers, preferably human keratin fibers, more preferably human hair.

14. Kit for hair comprising the composition according to claims 1 to 11.

## Patentansprüche

1. Kosmetische Zusammensetzung, **dadurch gekennzeichnet, dass** sie aufweist
a) ein Dünnschicht-bildendes Polymer, hergestellt mit den Monomeren, aufweisend Acrylsäure, Methacrylsäure und/oder Methacrylsäureester, wobei die Vernetzungsmittel aus Glycol-Dimethacrylat, Trimethylolpropantriacrylat, Trimethylolpropandiallylether, einzeln oder in einem beliebigen Gemisch davon, ausgewählt sind, vorzugsweise in einer Konzentration von 0,01 Gewichts-% bis 10 Gewichts-%, bezogen auf die Gesamtzusammensetzung,
b) ein oder mehrere Polyole in einer Konzentration von 8 Gewichts-% bis 18 Gewichts-%, bezogen auf die Gesamtzusammensetzung,
c) ein oder mehrere Aminosilikone,
wobei die Zusammensetzung ein oder mehrere zusätzliche(s) Dünnschicht-bildende(s) Polymer(e) aufweist, ausgewählt aus Polyvinylpyrrolidon-Copolymeren oder -Homopolymeren.

2. Zusammensetzung nach Anspruch 1 und/oder 2, **dadurch gekennzeichnet, dass** das Dünnschicht-bildende Polymer ein Polyvinylpyrrolidon-Homopolymer ist.

3. Zusammensetzung nach einem der vorhergehenden Ansprüche, **dadurch gekennzeichnet, dass** das Gewichtsverhältnis für die Verbindung aus a) und das zusätzliche Dünnschicht-bildende Polymer im Bereich von 1 bis 10, mehr bevorzugt von 2 bis 8 und am meisten bevorzugt von 2 bis 6 liegt.

4. Zusammensetzung nach einem der vorhergehenden Ansprüche, **dadurch gekennzeichnet, dass** die Polyole aus Molekülen ausgewählt sind, welche 2 oder mehr Hydroxygruppen aufweisen und eine lineare Kohlenstoffkettenlänge von C2 bis C8 oder eine verzweigte Kohlenstoffkettenlänge von C3 bis C8 aufweisen.

5. Zusammensetzung nach einem der vorhergehenden Ansprüche, **dadurch gekennzeichnet, dass** die Zusammensetzung Glycerin als das Polyol aufweist, vorzugsweise als das einzige Polyol.

6. Zusammensetzung nach einem der vorhergehenden Ansprüche, **dadurch gekennzeichnet, dass** das Aminosilikon ausgewählt ist aus:
a. einer Verbindung gemäß der allgemeinen Struktur 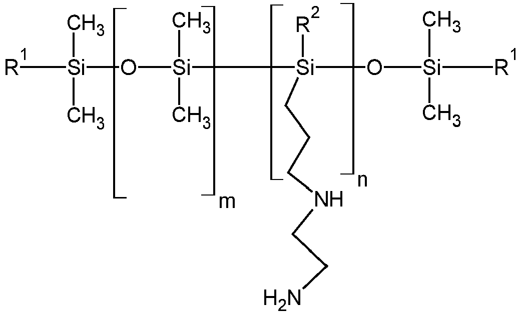 wobei R¹ aus OH, OCH₃ und/oder O-Si-(CH₃)₃ ausgewählt ist, R² aus CH₃, OCH₃, O-(Si-(CH₃)₂)ₓ-R¹ und/oder O-Si-(CH₃)₃ ausgewählt ist, mit der Maßgabe, dass, wenn R¹ oder R² aus O-Si-(CH₃)₃ ausgewählt ist, dann alle anderen R¹ oder R² aus O-Si-(CH₃)₃ und/oder OCH₃ ausgewählt sind,
b. einem Silikon-Pfropfcopolymer, aufweisend ein Organopolysiloxan-Segment als eine Hauptkette davon und ein aus einem ungesättigten Monomer abgeleitetes Polymersegment als eine Seitenkette davon, welches zu erhalten ist, indem zunächst ein Aminopropyldimethicon mit dem Thiolacton von Acylhomocystein umgesetzt wird und anschließend das so erhaltene mercapto-modifizierte Dimethicon mit einem Gemisch von N,N-Dimethylacrylamid und N-t-Butylacrylamid einer Pfropfcopolymerisation unterzogen wird,
c. einem Organopolysiloxan, wobei mindestens zwei Silicium-Atome in einem Organopolysiloxan-Segment, welches eine Hauptkette des Organopolysiloxans bildet, über eine Alkylengruppe, die ein Heteroatom enthält, an Poly(N-acylalkylenimin)-Segmente gebunden sind, die aus Wiederholungseinheiten bestehen, die durch die folgende allgemeine Formel (2) darzustellen sind: 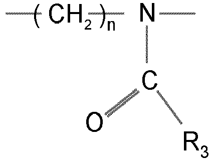 wobei R₃ ein Wasserstoffatom, eine Alkylgruppe mit 1 bis 22 Kohlenstoffatomen, eine Aralkylgruppe oder eine Arylgruppe ist; und n 2 oder 3 ist; wobei das Zahlenmittel-Molekulargewicht des Poly(N-acylalkylenimin)-Segments 1.200 bis 5.500 beträgt, wobei das Massenverhältnis der Organopolysiloxan-Segmente (a), welche die Hauptkette bilden, zu den Poly(N-acylalkylenimin)-Segmenten (b) [Le., alb] 35/65 bis 60/40 beträgt, wobei das Gewichtsmittel-Molekulargewicht der benachbarten Poly(N-acylalkylenimin)-Segmente 1.300 bis 5.500 beträgt und wobei das Gewichtsmittel-Molekulargewicht des Organopolysiloxan-Segments, das die Hauptkette bildet, 7.000 bis 100.000 beträgt.

7. Zusammensetzung nach einem der vorhergehenden Ansprüche, **dadurch gekennzeichnet, dass** sie eine wässrige Zusammensetzung mit einer Konzentration von weniger als 25 Gewichts-% an anderen organischen Lösungsmitteln als Polyolen ist, vorzugsweise mit weniger als 20 Gewichts-% an anderen als Polyolen und mehr bevorzugt mit weniger als 15 Gewichts-% an anderen als Polyolen, bezogen auf die Gesamtzusammensetzung.

8. Zusammensetzung nach einem der vorhergehenden Ansprüche, **dadurch gekennzeichnet, dass** sie ein oder mehrere Tenside aufweist, ausgewählt aus nicht-ionischen, kationischen, anionischen oder zwitterionischen oder Gemischen davon.

9. Zusammensetzung nach Anspruch 9, **dadurch gekennzeichnet, dass** sie ein oder mehrere nicht-ionische Tenside aufweist.

10. Zusammensetzung nach einem der vorhergehenden Ansprüche, **dadurch gekennzeichnet, dass** sie eine oder mehrere der Verbindungen aufweist, ausgewählt aus UV-Filtern, Antioxidationsmitteln, Konservierungsmitteln, Duftstoffen, Ceramiden, Ubichinonen.

11. Zusammensetzung nach einem der vorhergehenden Ansprüche, **dadurch gekennzeichnet, dass** die Viskosität der Zusammensetzung mehr als 10.000 mPas beträgt, gemessen mit einem Brookfield-Viskosimeter bei 25 °C.

12. Verwendung der Zusammensetzung nach Anspruch 1 bis 11 zum Styling von Keratinfasern, vorzugsweise menschlichen Keratinfasern, mehr bevorzugt menschlichen Haaren, und/oder zum Verringern des Kräuselns von Haaren.

13. Verfahren zum Styling von Haaren, **dadurch gekennzeichnet, dass** die in Anspruch 1 bis 11 definierte Zusammensetzung auf Keratinfasern, vorzugsweise menschliche Keratinfasern, mehr bevorzugt menschliche Haare aufgebracht wird.

14. Kit für Haare, welches die Zusammensetzung nach Anspruch 1 bis 11 aufweist.

## Revendications

1. Composition cosmétique **caractérisée en ce qu'**elle comprend
a) un polymère filmogène préparé avec les monomères comprenant de l'acide acrylique, de l'acide méthacrylique et/ou des esters d'acide méthacrylique, où les agents de réticulation sont choisis parmi le diméthacrylate de glycol, le triacrylate de triméthylol propane, le diallyl triméthylol propane éther, individuellement ou dans un mélange quelconque de ceux-ci, de préférence à une concentration dans la plage de 0,01 à 10 % en poids, calculée par rapport au total de la composition,
b) un ou plusieurs polyols à une concentration de 8 % à 18 % en poids, calculée par rapport au total de la composition,
c) une ou plusieurs amino silicones,
où la composition comprend un ou plusieurs polymère(s) filmogène(s) supplémentaire(s) choisi(s) parmi des copolymères ou des homopolymères de la polyvinylpyrrolidone.

2. Composition selon les revendications 1 et/ou 2, **caractérisée en ce que** polymère filmogène est un homopolymère de la polyvinylpyrrolidone.

3. Composition selon l'une quelconque des revendications précédentes, **caractérisée en ce que** le rapport en poids du composé a) et le polymère filmogène supplémentaire est dans la plage de 1 à 10, plus préférentiellement de 2 à 8, et idéalement de 2 à 6.

4. Composition selon l'une quelconque des revendications précédentes, **caractérisée en ce que** les polyols sont choisis parmi des molécules comprenant 2 ou plusieurs groupements hydroxyles et ayant une longueur de chaîne carbonée linéaire de C2 à C8 ou une longueur de chaîne carbonée ramifiée de C3 à C8.

5. Composition selon l'une quelconque des revendications précédentes, **caractérisée en ce que** la composition comprend de la glycérine servant de polyol, de préférence servant d'unique polyol.

6. Composition selon l'une quelconque des revendications précédentes, **caractérisée en ce que** l'amino silicone est choisie parmi :
a. un composé selon la structure générale 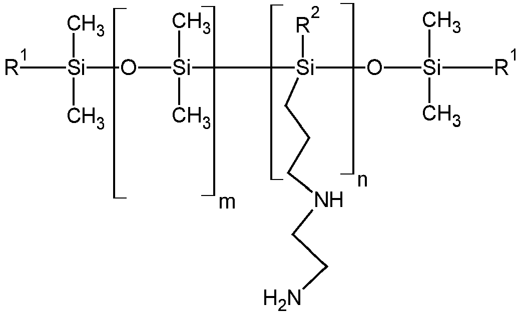 où R¹ est choisi parmi des groupements OH, OCH₃, et/ou O-Si-(CH₃)₃, R² est choisi parmi des groupements CH₃, OCH₃, O-(Si-(CH₃)₂)ₓ-R¹, et/ou O-Si-(CH₃)₃, à condition que, si R¹ ou R² sont choisis parmi un groupement O-Si-(CH₃)₃, alors tous les autres R¹ ou R² sont choisis parmi les groupements O-Si-(CH₃)₃ et/ou OCH₃,
b. un copolymère greffé silicone comprenant un segment organo polysiloxane lui servant de chaîne principale et un segment polymère dérivé d'un monomère insaturé lui servant de chaîne latérale, qui peut être obtenu en faisant réagir en premier lieu une aminopropyl diméthicone avec la thiolactone de l'acétyl homocystéine et en faisant une copolymérisation par greffage de la diméthicone modifiée mercapto ainsi obtenue avec un mélange de N,N-diméthylacrylamide et de N-t-butylacrylamide,
c. un organo polysiloxane, où au moins deux atomes de silicium dans un segment organo polysiloxane constituant une chaîne principale de l'organo polysiloxane sont liés à des segments poly(N-acylalkylène imine) constitués d'unités de répétition représentées par la formule générale (2) suivante via un hétéroatome portant un groupement alkylène : 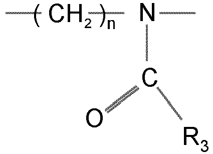 où R₃ est un atome d'hydrogène, un groupement alkyle ayant de 1 à 22 atomes de carbone, un groupement aralkyle, ou un groupement aryle ; et n est égal à 2 ou à 3 ; où la masse moléculaire moyenne en nombre du segment poly(N-acylalkylène imine) est de 1 200 à 5 500, où le rapport en masses des segments organo polysiloxane (a) constituant la chaîne principale sur les segments poly(N-acylalkylène imine) (b) [Le., alb] est de 35/65 à 60/40, où la masse moléculaire moyenne en poids des segments poly(N-acylalkylène imine) adjacents est de 1 300 à 5 500, et où la masse moléculaire moyenne en poids du segment organo polysiloxane constituant la chaine principale est de 7 000 à 100 000.

7. Composition selon l'une quelconque des revendications précédentes, **caractérisée en ce qu'**elle est une composition aqueuse avec une concentration inférieure à 25 % en poids de solvants organiques autres que des polyols, de préférence inférieure à 20 % en poids de solvants autres que des polyols, et plus préférentiellement inférieure à 15% en poids de solvants autres que des polyols, calculée par rapport au total de la composition.

8. Composition selon l'une quelconque des revendications précédentes, **caractérisée en ce qu'**elle comprend un ou plusieurs tensioactifs choisis parmi des tensioactifs non ioniques, cationiques, anioniques ou zwitterioniques, ou des mélanges de ceux-ci.

9. Composition selon la revendication 9, **caractérisée en ce qu'**elle comprend un ou plusieurs tensioactifs non ioniques.

10. Composition selon l'une quelconque des revendications précédentes, **caractérisée en ce qu'**elle comprend un ou plusieurs des composés choisis parmi des filtres UV, des antioxydants, des conservateurs, un parfum, des céramides, des ubiquinones.

11. Composition selon l'une quelconque des revendications précédentes, **caractérisée en ce que** la viscosité de la composition est supérieure à 10 000 mPa, mesurée avec un viscosimètre de Brookfield à 25 °C.

12. Utilisation de la composition selon les revendications 1 à 11 pour donner du style à des fibres de kératine, de préférence à des fibres de kératine humaines, plus préférentiellement à des cheveux humains, et/ou pour réduire la capacité des cheveux à former des frisottis.

13. Procédé pour donner du style aux cheveux, **caractérisé en ce que** la composition selon les revendications 1 à 11 est appliquée sur des fibres de kératine, de préférence des fibres de kératine humaine, plus préférentiellement des cheveux humains.

14. Kit pour cheveux comprenant la composition selon les revendications 1 à 11.
